(19) Europäisches Patentamt

European Patent Office

Office européen des brevets

(11) EP 1 122 534 A2

## (12) EUROPEAN PATENT APPLICATION

(43) Date of publication:
08.08.2001 Bulletin 2001/32

(51) Int Cl.⁷: G01N 21/64, A61B 5/00

(21) Application number: 01102606.9

(22) Date of filing: 06.02.2001

(84) Designated Contracting States:
AT BE CH CY DE DK ES FI FR GB GR IE IT LI LU MC NL PT SE TR
Designated Extension States:
AL LT LV MK RO SI

(30) Priority: 07.02.2000 JP 2000028568

(71) Applicant: Fuji Photo Film Co., Ltd.
Kanagawa-ken (JP)

(72) Inventor: Hakamata, Kazuo
Ashigarakami-gun, Kanagawa-ken (JP)

(74) Representative: Klunker . Schmitt-Nilson . Hirsch
Winzererstrasse 106
80797 München (DE)

### (54) Fluorescence imaging apparatus

(57) An excitation light irradiating device irradiates excitation light to a measuring site, the excitation light causing the measuring site to produce fluorescence. An imaging system detects an image of the fluorescence, which has been produced from the measuring site when the excitation light is irradiated to the measuring site. An imaging controller controls operations of the imaging system. The imaging system is provided with an image sensor, which comprises a plurality of pixels arrayed in two-dimensional directions and which has a fluorescence imaging region utilized for the imaging of the fluorescence and a non-imaging region other than the fluorescence imaging region. The imaging controller controls such that, when signal charges are to be read from the image sensor, signal charges, which have been accumulated in atleast certain pixels among pixels falling within the non-imaging region, are read with a quick reading operation, in which the signal charges are read at a reading speed higher than the reading speed for the fluorescence imaging region.

F I G . 1

EP 1 122 534 A2

## Description

BACKGROUND OF THE INVENTION

Field of the Invention

This invention relates to a fluorescence imaging apparatus for imaging fluorescence, which has been produced from a measuring site when excitation light is irradiated to the measuring site.

Description of the Related Art

It has heretofore been known that, in cases where excitation light having wavelengths falling within an excitation wavelength range for an intrinsic dye in a living body is irradiated to the living body, a fluorescence spectrum of fluorescence produced by the intrinsic dye in the living body varies for normal tissues and diseased tissues. Figure 9 shows typical fluorescence spectra of the fluorescence produced from normal tissues and the fluorescence produced from diseased tissues, which fluorescence spectra have been measured by the inventors. It is assumed that the thus produced fluorescence results from superposition of the fluorescence produced by various kinds of intrinsic dyes in the living body, such as FAD, collagen, fibronectin, and porphyrin.

There have heretofore been proposed systems wherein, by the utilization of the characteristics such that the fluorescence spectrum of the fluorescence produced by the intrinsic dye in the living body varies for the normal tissues and the diseased tissues, the fluorescence, which has been produced from a measuring site in a living body when the excitation light is irradiated to the measuring site, is imaged, a fluorescence image reflecting the intensity or the spectrum of the fluorescence is displayed on a monitor, and location and an infiltration range of the diseased tissues are thereby displayed as a change in color. In such systems, ordinarily, fluorescence imaging apparatuses for imaging the fluorescence, which has been produced from the measuring site in the living body when the excitation light is irradiated to the measuring site, are utilized.

However, the fluorescence imaging apparatuses have the problems in that the fluorescence, which is produced from the measuring site in the living body when the excitation light is irradiated to the measuring site, is weak and apt to be adversely affected by noise. In particular, a dark current occurring due to diffused current, and the like, constitutes noise depending upon a pixel size, an exposure time, a reading time, a temperature, and the like, and causes the signal-to-noise ratio of the fluorescence image to become low.

SUMMARY OF THE INVENTION

The primary object of the present invention is to provide a fluorescence imaging apparatus for imaging fluorescence, which has been produced from a measuring site when excitation light is irradiated to the measuring site, wherein a dark current occurring at the time of the imaging is reduced, and a fluorescence image having a high signal-to-noise ratio is capable of being obtained.

The present invention provides a first fluorescence imaging apparatus, comprising:

i) excitation light irradiating means for irradiating excitation light to a measuring site, the excitation light causing the measuring site to produce fluorescence,

ii) imaging means for imaging the fluorescence, which has been produced from the measuring site when the excitation light is irradiated to the measuring site, and

iii) imaging control means for controlling operations of the imaging means,

wherein the imaging means is provided with an image sensor, which comprises a plurality of pixels arrayed in two-dimensional directions and which has a fluorescence imaging region utilized for the imaging of the fluorescence and a non-imaging region other than the fluorescence imaging region, and the imaging control means controls such that, when signal charges are to be read from the image sensor, signal charges, which have been accumulated in at least certain pixels among pixels falling within the non-imaging region, are read with a quick reading operation, in which the signal charges are read at a reading speed higher than the reading speed for the fluorescence imaging region.

The present invention also provides a second fluorescence imaging apparatus, comprising:

i) excitation light irradiating means for irradiating excitation light to a measuring site, the excitation light causing the measuring site to produce fluorescence,

ii) imaging means for imaging the fluorescence, which has been produced from the measuring site when the excitation light is irradiated to the measuring site, and

iii) imaging control means for controlling operations of the imaging means,

wherein the imaging means is provided with an image sensor, which comprises a plurality of pixels arrayed in two-dimensional directions and which has a fluorescence imaging region utilized for the imaging of the fluorescence and a non-imaging region other than the fluorescence imaging region, and the imaging control means controls such that, when signal charges are to be read from the image sensor, signal charges, which have been accumulated in at least certain pixels among pixels falling within the non-imaging region, are read with a binning reading operation, in which the signal charges having been accumulated in a plurality of the pixels are added together, and a total sum signal charge having been obtained from the addition is read.

The present invention further provides a third fluorescence imaging apparatus, comprising:

i) excitation light irradiating means for irradiating excitation light to a measuring site, the excitation light causing the measuring site to produce fluorescence,
ii) imaging means for imaging the fluorescence, which has been produced from the measuring site when the excitation light is irradiated to the measuring site, and
iii) imaging control means for controlling operations of the imaging means,

wherein the imaging means is provided with an image sensor, which comprises a plurality of pixels arrayed in two-dimensional directions and which has a fluorescence imaging region utilized for the imaging of the fluorescence and a non-imaging region other than the fluorescence imaging region, and
the imaging control means controls such that, when signal charges are to be read from the image sensor, signal charges, which have been accumulated in at least certain pixels among pixels falling within the non-imaging region, are prevented from being read.

The present invention still further provides a fourth fluorescence imaging apparatus, comprising:

i) excitation light irradiating means for irradiating excitation light to a measuring site, the excitation light causing the measuring site to produce fluorescence,
ii) imaging means for imaging the fluorescence, which has been produced from the measuring site when the excitation light is irradiated to the measuring site, and
iii) imaging control means for controlling operations of the imaging means,

wherein the imaging means is provided with a charge transfer type of image sensor, which comprises a plurality of pixels arrayed in two-dimensional directions and which has a fluorescence imaging region utilized for the imaging of the fluorescence and a non-imaging region other than the fluorescence imaging region, and
the imaging control means controls such that, when signal charges are to be read from the image sensor, signal charges, which have been accumulated in pixels falling within a certain area of the non-imaging region, are read with either one of a quick reading operation, in which the signal charges are read at a reading speed higher than the reading speed for the fluorescence imaging region, and a binning reading operation, in which the signal charges having been accumulated in a plurality of the pixels are added together, and a total sum signal charge having been obtained from the addition is read, and signal charges, which have been accumulated in pixels falling within the other area of the non-imaging region, are prevented from being read.

In the third and fourth fluorescence imaging apparatuses in accordance with the present invention, the image sensor should preferably be provided with a clearing section for clearing signal charges, which have been accumulated in pixels.

Also, the third and fourth fluorescence imaging apparatuses in accordance with the present invention should preferably be modified such that the image sensor is provided with horizontal shifting means, from which the signal charges are read in one direction,

the imaging control means controls such that the signal charges having been accumulated in the pixels are transferred to the horizontal shifting means and are then read from the horizontal shifting means, and
the fluorescence imaging region is located at a position shifted from a center position on an imaging surface of the image sensor toward a side corresponding to a read-out side of the horizontal shifting means.

The present invention also provides a fifth fluorescence imaging apparatus, comprising:

i) excitation light irradiating means for irradiating excitation light to a measuring site, the excitation light causing the measuring site to produce fluorescence,
ii) imaging means for imaging the fluorescence, which has been produced from the measuring site when the excitation light is irradiated to the measuring site, and
iii) imaging control means for controlling operations of the imaging means,

wherein the imaging means is provided with a random access type of image sensor, which comprises a plurality of pixels arrayed in two-dimensional directions and which has a fluorescence imaging region utilized for the imaging of the fluorescence and a non-imaging region other than the fluorescence imaging region, and
the imaging control means controls such that, when signal charges are to be read from the image sensor, only the signal charges, which have been accumulated in pixels falling within the fluorescence imaging region, are read.

It is sufficient for the charge transfer type of image sensor described above to be an image sensor, in which signal charges of pixels are successively transferred in parallel and in one direction, and from which the signal charges are then read. No limitation is imposed upon the kind of the pixels of the charge transfer type of image sensor. By way of example, the pixels of the charge transfer type of image sensor may be constituted of photodiodes, charge coupled devices (CCD's), or the like.

Also, it is sufficient for the random access type of image sensor described above to be an image sensor, from which the signal charges of pixels are read randomly. No limitation is imposed upon the kind of the pixels of the random access type of image sensor. By way of example, the pixels of the random access type of image sensor may be constituted of photodiodes, metal oxide semiconductor (MOS) types of transistors, or the like.

With the first fluorescence imaging apparatus in accordance with the present invention, the signal charges, which have been accumulated in at least certain pixels among the pixels falling within the non-imaging region other than the fluorescence imaging region, are read with the quick reading operation, in which the signal charges are read at a reading speed higher than the reading speed for the fluorescence imaging region. Therefore, the time required to read the signal charges from the pixels of the non-imaging region is capable of being kept short. Accordingly, the reading time for the entire image sensor is capable of being kept short, the dark current depending upon the reading time is capable of being reduced, and the signal-to-noise ratio of the image formed with the imaging operation is capable of being enhanced.

With the second fluorescence imaging apparatus in accordance with the present invention, the signal charges, which have been accumulated in at least certain pixels among the pixels falling within the non-imaging region other than the fluorescence imaging region, are read with the binning reading operation, in which the signal charges having been accumulated in a plurality of the pixels are added together, and the total sum signal charge having been obtained from the addition is read. Therefore, the time required to read the signal charges from the pixels of the non-imaging region is capable of being kept short. Accordingly, the reading time for the entire image sensor is capable of being kept short, the dark current constituting noise contained in the image formed with the imaging operation is capable of being reduced, and the signal-to-noise ratio of the image formed with the imaging operation is capable of being enhanced.

With the third fluorescence imaging apparatus in accordance with the present invention, the signal charges, which have been accumulated in at least certain pixels among the pixels falling within the non-imaging region other than the fluorescence imaging region, are prevented from being read. Therefore, the time required to read the signal charges from the pixels of the non-imaging region is capable of being kept short. Accordingly, the reading time for the entire image sensor is capable of being kept short, the dark current constituting noise contained in the image formed with the imaging operation is capable of being reduced, and the signal-to-noise ratio of the image formed with the imaging operation is capable of being enhanced.

With the fourth fluorescence imaging apparatus in accordance with the present invention, the signal charges, which have been accumulated in pixels falling within the certain area of the non-imaging region other than the fluorescence imaging region of the charge transfer type of image sensor, are read with either one of the quick reading operation, in which the signal charges are read at a reading speed higher than the reading speed for the fluorescence imaging region, and the binning reading operation, in which the signal charges having been accumulated in a plurality of the pixels are added together, and the total sum signal charge having been obtained from the addition is read. Also, the signal charges, which have been accumulated in pixels falling within the other area of the non-imaging region, are prevented from being read. Therefore, only the signal charges having been accumulated in the pixels, which it is necessary to read in order for the signal charges having been accumulated in the pixels of the fluorescence imaging region to be read, are capable of being read. As a result, the time required to read the signal charges from the pixels of the non-imaging region is capable of being kept short. Accordingly, the reading time for the entire image sensor is capable of being kept short, the dark current constituting noise contained in the image formed with the imaging operation is capable of being reduced, and the signal-to-noise ratio of the image formed with the imaging operation is capable of being enhanced.

With the third and fourth fluorescence imaging apparatuses in accordance with the present invention, wherein the image sensor is provided with the clearing section for clearing signal charges, which have been accumulated in pixels, the signals charges remaining in the image sensor without being read are capable of being cleared easily.

Also, in cases where the charge transfer type of image sensor is employed as the image sensor, in order for the signal charges having been accumulated in the pixels of the fluorescence imaging region to be read, it is necessary for the signal charges having been accumulated in the pixels falling within the area of the non-imaging region, which area is located on the side of the imaging surface corresponding to the read-out side of the horizontal shifting means, to be read. However, the signal charges having been accumulated in the pixels falling within the area of the non-imaging region, which area is located on the opposite side, i.e. the side of the imaging surface opposite to the side corresponding to the read-out side of the horizontal shifting means, need not be read. Therefore, in cases where the fluorescence imaging region of the image sensor is located at a position shifted from the center position on the imaging surface of the image sensor toward the side corresponding to the read-out side of the horizontal shifting means, the number of the pixels falling within the area of the non-imaging region, from which pixels the signal charges should be read, becomes small. Accordingly, the reading time for the entire image sensor is capable of being minimized.

With the fifth fluorescence imaging apparatus in accordance with the present invention, only the signal charges having been accumulated in the pixels falling within the fluorescence imaging region of the random access type of image sensor are read. Therefore, the reading time for the entire image sensor is capable of being kept short, the dark current depending upon the reading time is capable of being reduced, and the signal-to-noise ratio of the image formed with the imaging operation is capable of being enhanced.

BRIEF DESCRIPTION OF THE DRAWINGS

Figure 1 is a schematic view showing an endoscope system, in which a first embodiment of the fluorescence imaging apparatus in accordance with the present invention is employed,
Figure 2 is a schematic view showing a CCD image sensor employed in the endoscope system, in which the first embodiment of the fluorescence imaging apparatus in accordance with the present invention is employed,
Figure 3 is an enlarged explanatory view showing part of the CCD image sensor of Figure 2,
Figure 4 is a schematic view showing a mosaic filter,
Figure 5 is an explanatory view showing signal charges having been transferred to a horizontal shift register,
Figure 6 is a schematic view showing a MOS type of image sensor employed in an endoscope system, in which a second embodiment of the fluorescence imaging apparatus in accordance with the present invention is employed,
Figure 7 is an enlarged explanatory view showing part of the MOS type of image sensor of Figure 6,
Figure 8 is a schematic view showing a CCD image sensor employed in an endoscope system, in which a third embodiment of the fluorescence imaging apparatus in accordance with the present invention is employed, and
Figure 9 is a graph showing spectral intensity distributions of fluorescence produced from normal tissues and fluorescence produced from diseased tissues.

DESCRIPTION OF THE PREFERRED EMBODIMENTS

The present invention will hereinbelow be described in further detail with reference to the accompanying drawings.

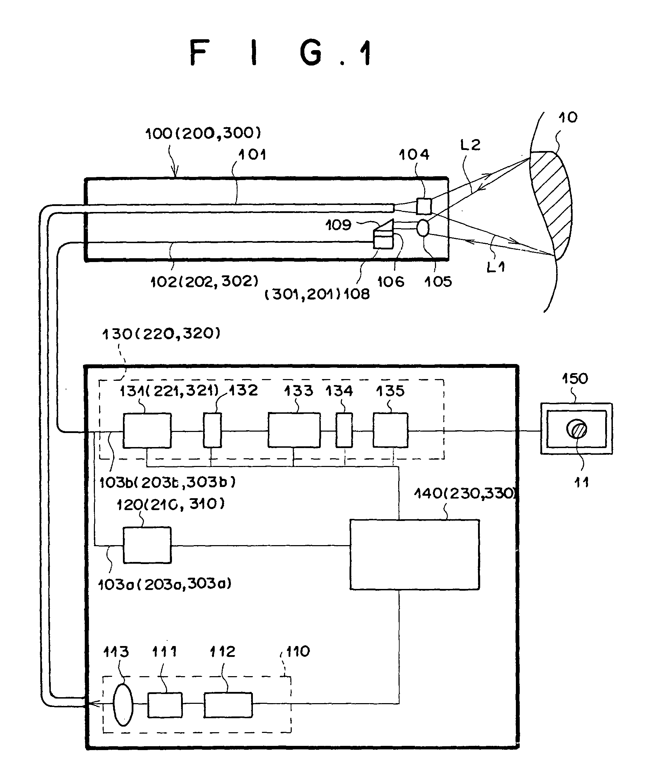

Firstly, an endoscope system, in which a first embodiment of the fluorescence imaging apparatus in accordance with the present invention is employed, will be described hereinbelow with reference to Figure 1 to Figure 5. Figure 1 is a schematic view showing the endoscope system, in which the first embodiment of the fluorescence imaging apparatus in accordance with the present invention is employed. In the endoscope system, in which the first embodiment of the fluorescence imaging apparatus in accordance with the present invention is employed, excitation light is irradiated to a measuring site in a living body, the excitation light causing the measuring site to produce fluorescence. The fluorescence produced from the measuring site is detected by a CCD image sensor, which is located at a leading end of an endoscope. The thus detected fluorescence image is displayed on a monitor and as a pseudo color image in accordance with a ratio between signal intensities of fluorescence components of the fluorescence, which fluorescence components have wavelengths falling within predetermined wavelength regions. When signal charges having been accumulated in the CCD image sensor, are to be read from the CCD image sensor, signal charges, which have been accumulated in pixels falling within a non-imaging region other than a fluorescence imaging region, are read with a quick reading operation, wherein the signal charges are read at a reading speed higher than the reading speed at which the signal charges having been accumulated in pixels falling within the fluorescence imaging region are read.

The endoscope system, in which the first embodiment of the fluorescence imaging apparatus in accordance with the present invention is employed, is provided with the CCD image sensor, which is a charge transfer type of image sensor, at the leading end of the endoscope. The endoscope system comprises an endoscope 100 to be inserted into a region of a patient, which region is considered as being a diseased part, and an illuminating unit 110 provided with a light source for producing the excitation light, which is used when an imaging operation for detecting the fluorescence image is to be performed. The endoscope system also comprises a CCD driver 120 for controlling the operations of the CCD image sensor. The endoscope system further comprises an image processing unit 130 for performing image processing for displaying the fluorescence image as a pseudo color image in accordance with the ratio between signal intensities of fluorescence components of the fluorescence, which fluorescence components have wavelengths falling within predetermined wavelength regions. The endoscope system still further comprises a controller 140, which controls operation timings. The endoscope system also comprises a monitor 150 for displaying the fluorescence image (specifically, the pseudo color image of the fluorescence image).

A light guide 101 and a CCD cable 102 extend in the endoscope 100 up to a leading end of the endoscope 100. An illuminating lens 104 is located at a leading end of the light guide 101, i.e. at the leading end of the endoscope 100. An objective lens 105 is located at a leading end of the CCD cable 102, i.e. at the leading end of the endoscope 100. A CCD image sensor 108 is connected to the leading end of the CCD cable 102. A mosaic filter 106, which comprises fine band-pass filters arrayed in a mosaic form, is combined with the CCD image sensor 108. Also, a prism 109 is mounted on the CCD image sensor 108.

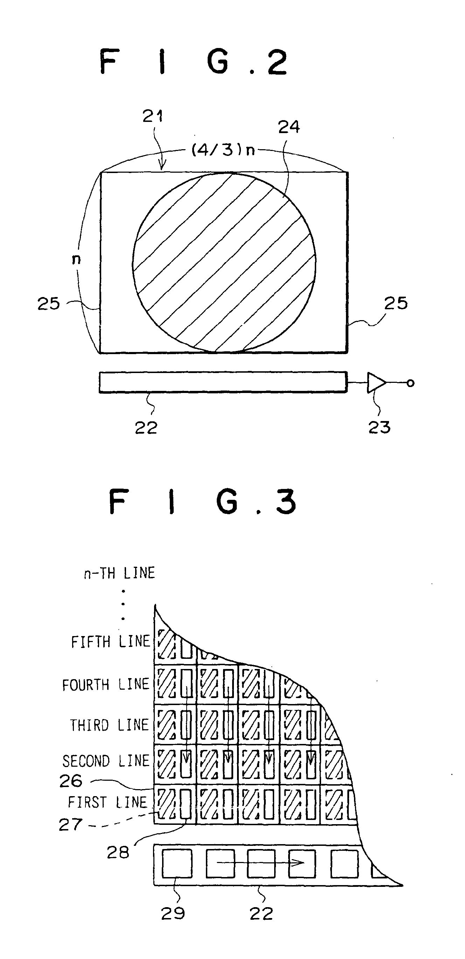

The CCD image sensor 108 is an interline type of CCD image sensor. As illustrated in Figure 2, the CCD image sensor 108 is provided with an imaging surface 21, which comprises an array of $n \times (4/3)n$ pixels having a square shape. The CCD image sensor 108 is also provided with a horizontal shift register 22 and an output circuit 23.

In the CCD image sensor 108, a region inward from a circle inscribed in the peripheral sides of the imaging surface 21 is a fluorescence imaging region 24, which is utilized for the imaging of the fluorescence. Areas outward from the fluorescence imaging region 24 in the imaging surface 21 are the areas forming a non-imaging region 25. The areas forming the non-imaging region 25 other than the fluorescence imaging region 24 are blocked by thin metal films, and the like. The area of the fluorescence imaging region 24 occupies 59% of the area of the imaging surface 21. The non-imaging region 25 occupies 41% of the area of the imaging surface 21.

Figure 3 is an enlarged explanatory view showing part of the CCD image sensor 108 of Figure 2. Each of pixels 26, 26, ... is constituted of a photodiode 27 for performing photoelectric conversion and a vertical transfer CCD 28 for performing vertical transfer of a signal charge. The horizontal shift register 22 is constituted of $(4/3)n$ number of horizontal transfer CCD's 29, 29, ...

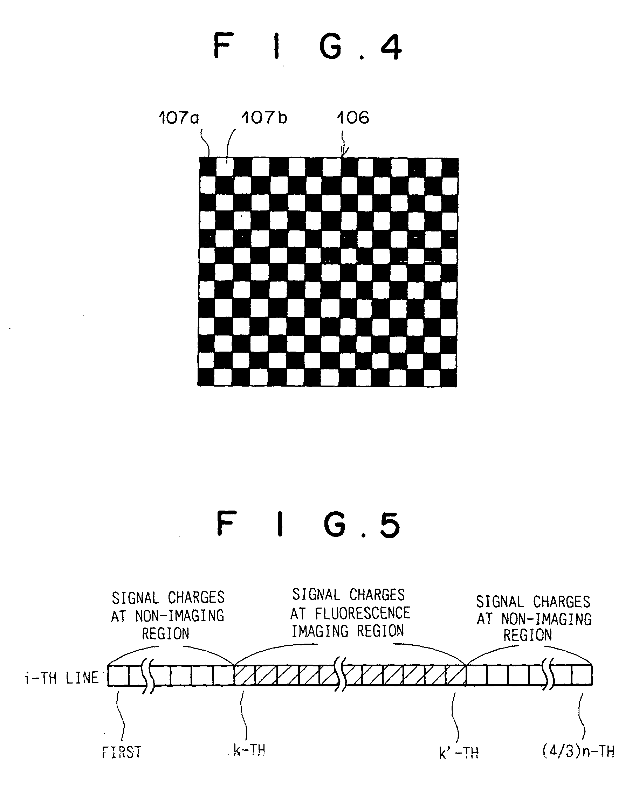

As illustrated in Figure 4, the mosaic filter 106 comprises blue band-pass filters 107a, 107a, ... and entire wavelength band-pass filters 107b, 107b, ..., which are arrayed alternately. The blue band-pass filters 107a, 107a, ... transmit only fluorescence components having wavelengths falling within a wavelength region of 430nm to 520nm. The entire wavelength band-pass filters 107b, 107b, ... transmit fluorescence components having wavelengths falling within a wavelength region of 430nm to 700nm. Each of the band-pass filters of the mosaic filter 106 corresponds to one of pixels in the CCD image sensor 108.

The light guide 101 is constituted of a quartz glass fiber and is connected to the illuminating unit 110. The CCD cable 102 comprises a driving line 103a, through which a driving signal for the CCD image sensor 108 is transmitted, and an output line 103b, through which the signal charges are read from the CCD image sensor 108. One end of the driving line 103a is connected to the CCD driver 120. One end of the output line 103b is connected to the image processing unit 130.

The illuminating unit 110 comprises a GaN type of semiconductor laser 111 for producing excitation light L1, which is used when the imaging operation for detecting the fluorescence image is to be performed, and an electric power source 112, which is electrically connected to the GaN type of semiconductor laser 111.

The image processing unit 130 comprises a signal processing circuit 131 for forming pseudo color image signals from the fluorescence image having been detected by the CCD image sensor 108. The image processing unit 130 also comprises an analog-to-digital converting circuit 132 for digitizing the pseudo color image signals, which have been obtained from the signal processing circuit 131. The image processing unit 130 further comprises a fluorescence image memory 133 for storing the digital pseudo color image signals, which have been obtained from the analog-to-digital converting circuit 132. The image processing unit 130 still further comprises a digital-to-analog converting circuit 134 for performing digital-to-analog conversion on the pseudo color image signals, which have been received from the fluorescence image memory 133. The image processing unit 130 also comprises a fluorescence image encoder 135 for transforming the pseudo color image signals, which have been received from the digital-to-analog converting circuit 134, into video signals. The controller 140 is connected to respective devices and controls the operation timings.

The CCD driver 120 constitutes the imaging control means of the fluorescence imaging apparatus in accordance with the present invention. In the CCD driver 120, information, which represents the locations of the fluorescence imaging region 24 and the non-imaging region 25 on the CCD image sensor 108, and reading control procedures have been stored previously.

How the endoscope system, in which the first embodiment of the fluorescence imaging apparatus in accordance with the present invention is employed, operates will be described hereinbelow.

The electric power source 112 for the GaN type of semiconductor laser 111 is driven in accordance with a control signal fed from the controller 140, and the excitation light L1 having a wavelength of 410nm is produced by the GaN type of semiconductor laser 111. The excitation light L1 passes through a lens 113 and impinges upon the light guide 101. The excitation light L1 is guided through the light guide 101 to the leading end of the endoscope 100, passes through the illuminating lens 104, and is irradiated to the measuring site 10.

When the measuring site 10 is exposed to the excitation light L1, the fluorescence L2 is produced from the measuring site 10. The fluorescence L2 is converged by the objective lens 105 and reflected by the prism 109. The fluorescence L2 then passes through the mosaic filter 106 and is received by the CCD image sensor 108.

In the CCD image sensor 108, the photodiodes 27, 27, ... of the pixels 26, 26, ... photoelectrically convert the incident fluorescence into electric signals in accordance with fluorescence intensities. After a predetermined length of time, the signal charges having been accumulated in the photodiodes 27, 27, ... are transferred simultaneously into the vertical transfer CCD's 28, 28, ..., which are adjacent to the photodiodes 27, 27, ... Thereafter, the vertical transfer CCD's 28, 28, ... transfer the signal charges in parallel and in the vertical direction. The signal charges, which have been transferred in the vertical direction, are successively fed into the horizontal transfer CCD's 29, 29, ... of the horizontal shift register 22.

In the horizontal shift register 22, when the signal charges of the pixels arrayed in one horizontal line have been received, the signal charges are transferred in the horizontal direction and are read via the output circuit 23, which is located at the right end. After all of the signal charges in the horizontal transfer CCD's 29, 29, ... have been read, the signal charges of the next horizontal line are transferred from the vertical transfer CCD's 28, 28, ... into the horizontal transfer CCD's 29, 29, ... The operations described above are iterated, and the signal charges having been accumulated in the pixels are read one after another, beginning with the signal charge of the pixel located at the right bottom of the imaging surface 21. After the signal charges of one horizontal line have been read, the signal charges of the next upper horizontal line are read. In this manner, the signal charges of all pixels, i.e. the signal charges corresponding to one image plane, are read.

How the reading operation for reading the signal charges from the horizontal shift register 22 is performed will be described hereinbelow with reference to Figure 5. Figure 5 is an explanatory view showing the signal charges of the pixels arrayed along an i-th line, which signal charges have been transferred to the horizontal shift register 22.

Specifically, the signal charges, which have been accumulated with the imaging with the fluorescence imaging region 24 of the imaging surface 21, have been transferred into the horizontal transfer CCD's 29, 29, ... ranging from the k-th horizontal transfer CCD 29 to the k'-th horizontal transfer CCD 29, which are represented by the formulas shown below.

$$k = (2/3)n - \sqrt{ni\text{-}i^2} - 1$$

$$k' = (2/3)n + \sqrt{ni\text{-}i^2} + 1$$

Also, the signal charges, which have been accumulated with the imaging with the left area of the non-imaging region 25 of the imaging surface 21, have been transferred into the horizontal transfer CCD's 29, 29, ... ranging from the first horizontal transfer CCD 29 at the left end to the (k-1)-th horizontal transfer CCD 29. Further, the signal charges, which have been accumulated with the imaging with the right area of the non-imaging region 25 of the imaging surface 21, have been transferred into the horizontal transfer CCD's 29, 29, ... ranging from the (k'+1) - th horizontal transfer CCD 29 to the (4/3)n-th horizontal transfer CCD 29 at the right end.

The signal charges necessary for the displaying of the fluorescence image are only the signal charges, which have been transferred into the horizontal transfer CCD's 29, 29, ... ranging from the k-th horizontal transfer CCD 29 to the k'-th horizontal transfer CCD 29. The signal charges having been transferred into the other horizontal transfer CCD's 29, 29, ... are the unnecessary signal charges.

Ordinarily, in cases where the signal charges are to be read from the horizontal shift register 22 via the output circuit 23, the signal charges are read at a predetermined speed, such that noise may be prevented from occurring. However, in cases where the unnecessary signal charges are to be read, the occurrence of noise need not be taken into consideration, and therefore the reading speed is capable of being set at a high speed.

In the CCD driver 120, the information, which represents the locations of the fluorescence imaging region 24 and the non-imaging region 25 on the imaging surface 21, and the information, which represents the values of k and k' corresponding to each line, have been stored previously. The CCD driver 120 controls such that, when the signal charges of the i-th line are to be read from the horizontal shift register 22, the signal charges having been transferred into the horizontal transfer CCD's 29, 29, ... ranging from the k-th horizontal transfer CCD 29 to the k'-th horizontal transfer CCD 29 are read at the predetermined speed. Also, the CCD driver 120 controls such that, when the signal charges of the i-th line are to be read from the horizontal shift register 22, the signal charges, which have been transferred into the horizontal transfer CCD's 29, 29, ... ranging from the first horizontal transfer CCD 29 at the left end to the (k-1) - th horizontal transfer CCD 29, and the signal charges, which have been transferred into the horizontal transfer CCD's 29, 29, ... ranging from the (k'+1) - th horizontal transfer CCD 29 to the (4/3)n-th horizontal transfer CCD 29 at the right end, are read at a speed 10 times as high as the predetermined speed described above.

In the signal processing circuit 131, the processes, such as correlative double sampling, clamping, blanking, and amplification, are performed on the signals, which have been read from the pixels 26, 26, ... falling within the fluorescence imaging region 24, among the signals having been obtained from the CCD image sensor 108. Thereafter, with respect to each pixel pair, a signal intensity B2 of the fluorescence components of the fluorescence L2, which fluorescence components have wavelengths falling within the blue wavelength region and have passed through the blue band-pass filters 107a, 107a, ..., and a signal intensity W2 of the fluorescence components of the fluorescence L2, which fluorescence components have wavelengths falling within the entire measurement wavelength region and have passed through the entire wavelength band-pass filters 107b, 107b, ..., are detected. Also, with respect to each pixel pair, color difference matrix operations according to an NTSC method are performed by utilizing the signal intensity B2 and the signal intensity W2. In this manner, a pseudo luminance signal Y2 and pseudo color difference signals R2-Y2 and B2-Y2, which act as the pseudo color image signals, are calculated.

The pseudo color image signals (i.e., the pseudo luminance signal Y2 and the pseudo color difference signals R2-Y2 and B2-Y2), which are made up of pseudo color image signal components corresponding to respective pixels and have been obtained from the signal processing circuit 131, are digitized by the analog-to-digital converting circuit 132. The thus obtained pseudo color image signals are stored in the fluorescence image memory 133. In accordance with the display timing, the pseudo color image signals are read from the fluorescence image memory 133 and are subjected to the digital-to-analog conversion in the digital-to-analog converting circuit 134. The pseudo color image signals are then transformed by the fluorescence image encoder 135 into predetermined video signals. The thus obtained video signals are fed from the fluorescence image encoder 135 into the monitor 150 and utilized for displaying a fluorescence image 11.

The fluorescence image 11 is displayed with a pseudo color, such that the display color varies in accordance with the ratio between the signal intensity W2 of the fluorescence components, which have wavelengths falling within the entire measurement wavelength region, and the signal intensity B2 of the fluorescence components, which have wavelengths falling within the blue wavelength region. The tint of the pseudo color of the fluorescence image 11 is determined by coefficients in matrix operation formulas employed in the signal processing circuit 131.

The coefficients described above should preferably be selected such that the difference between the display color for the fluorescence, which has been produced from the normal tissues, and the display color for the fluorescence, which has been produced from the diseased tissues, may be clear. For example, the pseudo color may be displayed by selecting the coefficients such that the fluorescence, which has been produced from the normal tissues, may be displayed in white, and the fluorescence, which has been produced from the diseased tissues, may be displayed in pink or in one of other colors. In such cases, the person, who sees the displayed image, is capable of easily recognizing the state of the diseased tissues.

As described above, the signal charges, which have been accumulated in the pixels falling within the non-imaging region 25 other than the fluorescence imaging region 24 of the CCD image sensor 108 acting as the charge transfer type of image sensor, are read with the quick reading operation, in which the signal charges are read at a reading speed higher than the reading speed for the fluorescence imaging region 24. Therefore, the time required to read the signal charges from all of the pixels of the CCD image sensor 108 is capable of being kept short. Accordingly, the dark current depending upon the reading time is capable of being reduced, and the signal-to-noise ratio of the image formed with the imaging operation is capable of being enhanced.

In the endoscope system, in which the first embodiment of the fluorescence imaging apparatus in accordance with the present invention is employed, the signal charges, which have been accumulated in the pixels falling within the non-imaging region 25 other than the fluorescence imaging region 24, are read with the quick reading operation. In a modification of the first embodiment, the signal charges, which have been accumulated in the pixels falling within the non-imaging region 25 other than the fluorescence imaging region 24, may be read with a binning reading operation, in which the signal charges having been accumulated in a plurality of the pixels are added together, and the total sum signal charge having been obtained from the addition is read. In the modification of the first embodiment, the CCD driver 120 controls the reading of the signal charges from the horizontal shift register 22 such that, in cases where the signal charges, which have been accumulated in the pixels falling within the non-imaging region 25 and have then been transferred into the horizontal transfer CCD's 29, 29, ..., i.e. the signal charges, which have been transferred into the horizontal transfer CCD's 29, 29, ... ranging from the first horizontal transfer CCD 29 at the left end to the (k-1)-th horizontal transfer CCD 29 illustrated in Figure 5, and the signal charges, which have been transferred into the horizontal transfer CCD's 29, 29, ... ranging from the (k'+1)-th horizontal transfer CCD 29 to the (4/3)n-th horizontal transfer CCD 29 at the right end in Figure 5, are to be read, the signal charges in each group of four horizontal transfer CCD's 29, 29, 29, 29 are added together, and thereafter the total sum signal charge having been obtained from each of the additions is read.

For example, if the time required to read the signal charges from four horizontal transfer CCD's 29, 29, 29, 29 with the ordinary reading operation is taken as 4, the signal charges in the four horizontal transfer CCD's 29, 29, 29, 29 will be capable of being read within a time of approximately 1.1 with the binning reading operation. Therefore, the time required to read the signal charges from all of the pixels of the CCD image sensor 108 is capable of being kept short. Accordingly, the dark current depending upon the reading time is capable of being reduced, and the signal-to-noise ratio of the image formed with the imaging operation is capable of being enhanced. Alternatively, all of the signal charges, which have been transferred into the horizontal transfer CCD's 29, 29, ... ranging from the first horizontal transfer CCD 29 at the left end to the (k-1)-th horizontal transfer CCD 29, and the signal charges, which have been transferred into the horizontal transfer CCD's 29, 29, ... ranging from the (k'+1)-th horizontal transfer CCD 29 to the (4/3)n-th horizontal transfer CCD 29 at the right end, may be added together, and thereafter the total sum signal charge having been obtained from the addition may be read. In such cases, the reading time is capable of being minimized.

The quick reading operation or the binning reading operation need not necessarily be performed with respect to the entire non-imaging region 25 and may be performed with respect to only desired areas of the non-imaging region 25. Also, the quick reading operation may be performed with respect to a certain area of the non-imaging region 25, and the binning reading operation may be performed with respect to one of the other areas of the non-imaging region 25.

An endoscope system, in which a second embodiment of the fluorescence imaging apparatus in accordance with the present invention is employed, will be described hereinbelow. The constitution of the endoscope system, in which the second embodiment of the fluorescence imaging apparatus in accordance with the present invention is employed, is approximately identical with the constitution of the endoscope system, in which the first embodiment of the fluorescence imaging apparatus described above is employed. Therefore, only different elements are numbered with reference numerals in parentheses in Figure 1.

In the endoscope system, in which the second embodiment of the fluorescence imaging apparatus in accordance with the present invention is employed, the excitation light is irradiated to a measuring site in a living body, the excitation light causing the measuring site to produce the fluorescence. The fluorescence produced from the measuring site is detected by a MOS type of image sensor, which is located at a leading end of an endoscope. The thus detected fluorescence image is displayed on the monitor and as a pseudo color image in accordance with the ratio between the signal intensities of the fluorescence components of the fluorescence, which fluorescence components have wavelengths falling within predetermined wavelength regions. When the signal charges having been accumulated in the MOS type of image sensor, are to be read from the MOS type of image sensor, only the signal charges, which have been accumulated in the pixels falling within a fluorescence imaging region, are read.

The endoscope system, in which the second embodiment of the fluorescence imaging apparatus in accordance with the present invention is employed, is provided with the MOS type of image sensor at the leading end of the endoscope. The endoscope system comprises an endoscope 200 to be inserted into a region of a patient, which region is considered as being a diseased part, and the illuminating unit 110 provided with a light source for producing the excitation light. The endoscope system also comprises a driver 210 for controlling the operations of the MOS type of image sensor. The endoscope system further comprises an image processing unit 220 for performing the image processing for displaying the fluorescence image as a pseudo color image in accordance with the ratio between signal intensities of fluorescence components of the fluorescence, which fluorescence components have wavelengths falling within predetermined wavelength regions. The endoscope system still further comprises a controller 230, which controls operation timings. The endoscope system also comprises the monitor 150 for displaying the fluorescence image.

The light guide 101 and a cable 202 extend in the endoscope 200 up to a leading end of the endoscope 200. A MOS type of image sensor 201 is connected to the leading end of the cable 202. The mosaic filter 106 is combined with the MOS type of image sensor 201. Also, the prism 109 is mounted on the MOS type of image sensor 201. Each of the band-pass filters of the mosaic filter 106 corresponds to one of pixels in the MOS type of image sensor 201.

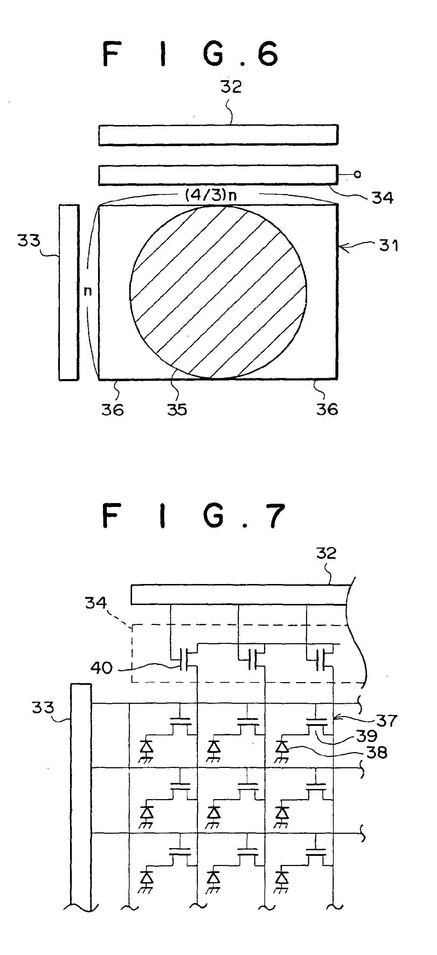

As illustrated in Figure 6, the MOS type of image sensor 201 is provided with an imaging surface 31, which comprises an array of $n\times(4/3)n$ pixels having a square shape. The MOS type of image sensor 201 is also provided with a horizontal scanning shift register 32, a vertical scanning shift register 33, and an output section 34. The MOS type of image sensor 201 is a random access type of image sensor.

In the MOS type of image sensor 201, a region inward from a circle inscribed in the peripheral sides of the imaging surface 31 is a fluorescence imaging region 35, which is utilized for the imaging of the fluorescence. Areas outward from the fluorescence imaging region 35 in the imaging surface 31 are the areas forming a non-imaging region 36. The areas forming the non-imaging region 36 other than the fluorescence imaging region 35 are blocked by thin metal films, and the like. The area of the fluorescence imaging region 35 occupies 59% of the area of the imaging surface 31. The non-imaging region 36 occupies 41% of the area of the imaging surface 31.

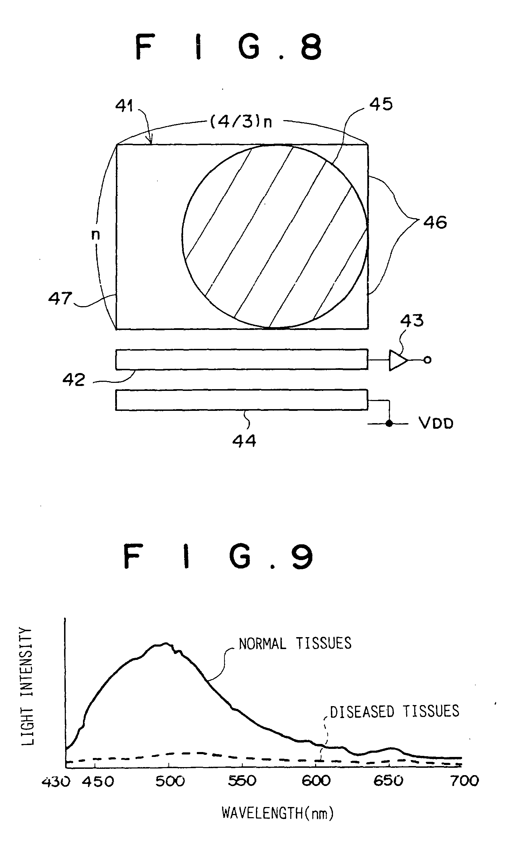

Figure 7 is an enlarged explanatory view showing part of the MOS type of image sensor 201 of Figure 6. Each of pixels 37, 37, ... is constituted of a photodiode 38 for performing photoelectric conversion and a MOS transistor 39 for performing switching operations. The MOS transistor 39 of each of the pixels 37, 37, ... is connected to the horizontal scanning shift register 32 and the vertical scanning shift register 33. The MOS transistor 39 is switched by an applied pulse so as to output the signal charge, which has been accumulated in the photodiode 38 of each pixel 37, to the output section 34. The output section 34 is constituted of (4/3)n number of output MOS transistors 40, 40, ...

The cable 202 comprises a driving line 203a, through which a driving signal for the MOS type of image sensor 201 is transmitted, and an output line 203b, through which the signals having been read from the MOS type of image sensor 201 are transmitted. One end of the driving line 203a is connected to the driver 210. One end of the output line 203b is connected to a signal processing circuit 221 for forming pseudo color image signals. The controller 230 is connected to respective devices and controls the operation timings.

The driver 210 constitutes the imaging control means of the fluorescence imaging apparatus in accordance with the present invention. In the driver 210, information, which represents the locations of the fluorescence imaging region 35 and the non-imaging region 36 on the MOS type of image sensor 201, and reading control procedures have been stored previously.

How the endoscope system, in which the second embodiment of the fluorescence imaging apparatus in accordance with the present invention is employed, operates will be described hereinbelow.

The excitation light L1 is irradiated to the measuring site 10 in accordance with a control signal fed from the controller 230. When the measuring site 10 is exposed to the excitation light L1, the fluorescence L2 is produced from the measuring site 10. The fluorescence L2 is converged by the objective lens 105 and reflected by the prism 109. The fluorescence L2 then passes through the mosaic filter 106 and is received by the MOS type of image sensor 201.

In the MOS type of image sensor 201, the photodiodes 38, 38, ... of the pixels 37, 37, ... photoelectrically convert the incident fluorescence into electric signals in accordance with the fluorescence intensities.

When the signal charges are to be read from the MOS type of image sensor 201, firstly, one of the horizontal lines is selected by the vertical scanning shift register 33. Thereafter, pulses are successively applied from the horizontal scanning shift register 32, and the MOS transistors 39, 39, ... of the pixels 37, 37, ... arrayed in the horizontal direction are switched successively from the left toward the right. In this manner, the signal charges having been accumulated in the photodiodes 38, 38, ... are read successively.

The driver 210 controls such that, when the signal charges are to be read from the MOS type of image sensor 201, the pulses are applied via the horizontal scanning shift register 32 and the vertical scanning shift register 33 only to the pixels falling within the fluorescence imaging region 35 in order to read the signal charges from the pixels, and no pulse is applied to the pixels falling within the non-imaging region 36 so as not to read the signal charges from the pixels.

In the signal processing circuit 221, the processes, such as correlative double sampling, clamping, blanking, and amplification, are performed on the signals, which have been read from the pixels 37, 37, ... falling within the fluorescence imaging region 35 on the MOS type of image sensor 201. Thereafter, with respect to each pixel pair, the signal intensity B2 of the fluorescence components of the fluorescence L2, which fluorescence components have wavelengths falling within the blue wavelength region and have passed through the blue band-pass filters 107a, 107a, ..., and the signal intensity W2 of the fluorescence components of the fluorescence L2, which fluorescence components have wavelengths falling within the entire measurement wavelength region and have passed through the entire wavelength band-pass filters 107b, 107b, ..., are detected. Also, with respect to each pixel pair, the color difference matrix operations according to the NTSC method are performed by utilizing the signal intensity B2 and the signal intensity W2. In this manner, the pseudo luminance signal Y2 and the pseudo color difference signals R2-Y2 and B2-Y2, which act as the pseudo color image signals, are calculated. Thereafter, the operations are performed in the same manner as that in the first embodiment described above, and the fluorescence image 11 is displayed on the monitor 150 and with a pseudo color, such that the display color varies in accordance with the ratio between the signal intensity W2 of the fluorescence components, which have wavelengths falling within the entire measurement wavelength region, and the signal intensity B2 of the fluorescence components, which have wavelengths falling within the blue wavelength region.

As described above, in the second embodiment, only the signal charges, which have been accumulated in the pixels 37, 37, ... falling within the fluorescence imaging region 35 that occupies 59% of the area of the imaging surface 31 of the MOS type of image sensor 201 acting as the random access type of image sensor, are read, and the signal charges, which have been accumulated in the pixels 37, 37, ... falling within the non-imaging region 36, are prevented from being read. Therefore, the reading time is capable of being reduced to approximately 60% of the reading time, which is required when the signal charges having been accumulated in all of the pixels 37, 37, ... of the MOS type of image sensor 201 are read. Accordingly, the dark current depending upon the reading time is capable of being reduced, and the signal-to-noise ratio of the image formed with the imaging operation is capable of being enhanced.

An endoscope system, in which a third embodiment of the fluorescence imaging apparatus in accordance with the present invention is employed, will be described hereinbelow. The constitution of the endoscope system, in which the third embodiment of the fluorescence imaging apparatus in accordance with the present invention is employed, is approximately identical with the constitution of the endoscope system, in which the first embodiment of the fluorescence imaging apparatus described above is employed. Therefore, only different elements are numbered with reference numerals in parentheses in Figure 1.

In the endoscope system, in which the third embodiment of the fluorescence imaging apparatus in accordance with the present invention is employed, the excitation light is irradiated to a measuring site in a living body, the excitation light causing the measuring site to produce fluorescence. The fluorescence produced from the measuring site is detected by a CCD image sensor, which is located at a leading end of an endoscope. The thus detected fluorescence image is displayed on a monitor and as a pseudo color image in accordance with a ratio between signal intensities of fluorescence components of the fluorescence, which fluorescence components have wavelengths falling within predetermined wavelength regions. When the signal charges having been accumulated in the CCD image sensor, are to be read from the CCD image sensor, the signal charges, which have been accumulated in pixels falling within a predetermined area of a non-imaging region other than a fluorescence imaging region, are read with the quick reading operation, wherein the signal charges are read at a reading speed higher than the reading speed at which the signal charges having been accumulated in pixels falling within the fluorescence imaging region are read, and the signal charges, which have been accumulated in pixels falling within the other area of the non-imaging region, are prevented from being read.

The endoscope system, in which the third embodiment of the fluorescence imaging apparatus in accordance with the present invention is employed, is provided with the CCD image sensor, which is a charge transfer type of image sensor, at the leading end of the endoscope. The endoscope system comprises an endoscope 300 to be inserted into a region of a patient, which region is considered as being a diseased part, and the illuminating unit 110 provided with a light source for producing the excitation light. The endoscope system also comprises a CCD driver 310 for controlling the operations of the CCD image sensor. The endoscope system further comprises an image processing unit 320 for performing the image processing for displaying the fluorescence image as a pseudo color image in accordance with the ratio between signal intensities of fluorescence components of the fluorescence, which fluorescence components have wavelengths falling within predetermined wavelength regions. The endoscope system still further comprises a controller 330, which controls operation timings. The endoscope system also comprises the monitor 150 for displaying the fluorescence image.

The light guide 101 and a cable 302 extend in the endoscope 300 up to a leading end of the endoscope 300. A CCD image sensor 301 is connected to the leading end of the cable 302. The mosaic filter 106 is combined with the CCD image sensor 301. Also, the prism 109 is mounted on the CCD image sensor 301. Each of the band-pass filters of the mosaic filter 106 corresponds to one of pixels in the CCD image sensor 301.

The CCD image sensor 301 is the interline type of CCD image sensor. As illustrated in Figure 8, the CCD image sensor 301 is provided with an imaging surface 41, which comprises an array of $n \times (4/3)n$ pixels having a square shape. The CCD image sensor 301 is also provided with a horizontal shift register 42 and an output circuit 43. The CCD image sensor 301 is further provided with a clearing drain 44, which is connected to an electric power source line.

In the CCD image sensor 301, a region inward from a circle inscribed in the upper, lower, and right sides of the imaging surface 41 is a fluorescence imaging region 45, which is utilized for the imaging of the fluorescence. An area located on the upper right side outward from the fluorescence imaging region 45 and an area located on the lower right side outward from the fluorescence imaging region 45 constitute a non-imaging region 46. Also, an area located on the left side outward from the fluorescence imaging region 45 constitutes a non-imaging region 47. The areas forming the non-imaging regions 46 and 47 other than the fluorescence imaging region 45 are blocked by thin metal films, and the like. The area of the fluorescence imaging region 45 occupies 59% of the area of the imaging surface 41. The non-imaging region 46 occupies 8% of the area of the imaging surface 41. Also, the non-imaging region 47 occupies 33% of the area of the imaging surface 41.

The horizontal shift register 42 is provided with $(4/3)n$ number of horizontal shift CCD's (not shown). Signal charges, which have been prevented from being read at the time of the signal charge reading operation and remain in the horizontal shift CCD's, are shifted to the clearing drain 44.

The cable 302 comprises a driving line 303a, through which a driving signal for the CCD image sensor 301 is transmitted, and an output line 303b, through which the signals having been read from the CCD image sensor 301 are transmitted. One end of the driving line 303a is connected to the CCD driver 310. One end of the output line 303b is connected to a signal processing circuit 321 for forming the pseudo color image signals. The controller 330 is connected to respective devices and controls the operation timings.

The CCD driver 310 constitutes the imaging control means of the fluorescence imaging apparatus in accordance with the present invention. In the CCD driver 310, information, which represents the locations of the fluorescence imaging region 45 and the non-imaging regions 46 and 47 on the CCD image sensor 301, and reading control procedures have been stored previously.

How the endoscope system, in which the third embodiment of the fluorescence imaging apparatus in accordance with the present invention is employed, operates will be described hereinbelow.

The excitation light L1 is irradiated to the measuring site 10 in accordance with a control signal fed from the controller 330. When the measuring site 10 is exposed to the excitation light L1, the fluorescence L2 is produced from the measuring site 10. The fluorescence L2 is converged by the objective lens 105 and reflected by the prism 109. The fluorescence L2 then passes through the mosaic filter 106 and is received by the CCD image sensor 301.

In the pixels of the CCD image sensor 301, the incident fluorescence is photoelectrically converted into electric signals in accordance with fluorescence intensities.

When the signal charges are to be read from the CCD image sensor 301, under the control of the CCD driver 310, the signal charges having been accumulated in the pixels arrayed along one horizontal line are transferred into the horizontal shift register 42. In the horizontal shift register 42, the signal charges, which have been accumulated in the pixels falling within the non-imaging region 46, are read at a reading speed 10 times as high as an ordinary reading speed. The signal charges, which have been accumulated in the pixels falling within the fluorescence imaging region 45, are read at the ordinary reading speed. Also, the signal charges, which have been accumulated in the pixels falling within the non-imaging region 47, are prevented from being read and remain in the horizontal shift register 42.

In this state, the signal charges, which have been accumulated in the pixels arrayed along the next horizontal line, are transferred into the horizontal shift register 42. At this time, the signal charges, which have been accumulated in the pixels falling within the non-imaging region 47 and remain in the horizontal shift register 42, are shifted to the clearing drain 44 and are cleared together from the clearing drain 44 to the electric power source line.

In the signal processing circuit 321, the processes, such as correlative double sampling, clamping, blanking, and amplification, are performed on the signals, which have been read from the pixels falling within the fluorescence imaging region 45 of the CCD image sensor 301. Thereafter, with respect to each pixel pair, the signal intensity B2 of the fluorescence components of the fluorescence L2, which fluorescence components have wavelengths falling within the blue wavelength region and have passed through the blue band-pass filters 107a, 107a, ..., and the signal intensity W2 of the fluorescence components of the fluorescence L2, which fluorescence components have wavelengths falling within the entire measurement wavelength region and have passed through the entire wavelength band-pass filters 107b, 107b, ..., are detected. Also, with respect to each pixel pair, the color difference matrix operations according to the NTSC method are performed by utilizing the signal intensity B2 and the signal intensity W2. In this manner, the pseudo luminance signal Y2 and the pseudo color difference signals R2-Y2 and B2-Y2, which act as the pseudo color image signals, are calculated.

Thereafter, the operations are performed in the same manner as that in the first embodiment described above, and the fluorescence image 11 is displayed on the monitor 150 and with a pseudo color, such that the display color varies in accordance with the ratio between the signal intensity W2 of the fluorescence components, which have wavelengths falling within the entire measurement wavelength region, and the signal intensity B2 of the fluorescence components, which have wavelengths falling within the blue wavelength region.

As described above, in the third embodiment, the signal charges, which have been accumulated in the pixels falling within the non-imaging region 46 that occupies 8% of the area of the imaging surface 41 of the CCD image sensor 301, are read with the quick reading operation, and the signal charges, which have been accumulated in the pixels falling within the non-imaging region 47 that occupies 33% of the area of the imaging surface 41 of the CCD image sensor 301, are prevented from being read. Therefore, the reading time is capable of being shorter than when the signal charges having been accumulated in all of the pixels of the CCD image sensor 301 are read. Accordingly, the dark current depending upon the reading time is capable of being reduced, and the signal-to-noise ratio of the image formed with the imaging operation is capable of being enhanced.

In the endoscope system, in which the third embodiment of the fluorescence imaging apparatus in accordance with the present invention is employed, the fluorescence imaging region 45 of the CCD image sensor 301 is in contact with the right side of the imaging surface 41. However, the fluorescence imaging region 45 need not necessarily be in contact with the right side of the imaging surface 41. It is sufficient for the fluorescence imaging region 45 to be located at a position shifted from the center position on the imaging surface 41 toward the right side, i.e. toward the side corresponding to the output side of the horizontal shift register 42. In such cases, the non-imaging region 47, for which the signal charge reading need not be performed, becomes wider and the reading time is capable of being kept shorter than when the fluorescence imaging region 45 is located at the center position on the imaging surface 41.

Also, in the endoscope system, in which the third embodiment of the fluorescence imaging apparatus in accordance with the present invention is employed, the signal charges, which have been accumulated in the pixels falling within the non-imaging region 46 of the CCD image sensor 301, are read with the quick reading operation, and the signal charges, which have been accumulated in the pixels falling within the non-imaging region 47, are cleared. In a modification of the third embodiment, the signal charges, which have been accumulated in the pixels falling within the non-imaging region 46, may be read with the binning reading operation, in which the signal charges having been accumulated in a plurality of the pixels are added together, and the total sum signal charge having been obtained from the addition is read. Also, the quick reading operation or the binning reading operation need not necessarily be performed with respect to the entire non-imaging region 46 and may be performed with respect to only desired areas of the non-imaging region 46.

Further, the quick reading operation or the binning reading operation may be performed with respect to a certain area of the non-imaging region 47. Furthermore, the quick reading operation may be performed with respect to certain areas of the non-imaging region 46 and the non-imaging region 47, and the binning reading operation may be performed with respect to other areas of the non-imaging region 46 and the non-imaging region 47.

The operation for clearing the signal charges need not necessarily be performed with respect to the entire area of the non-imaging region 47 and may be performed with respect to only desired areas of the non-imaging region 47. Specifically, it is sufficient for the signal charges, which have been prevented from being read with the reading operation and remain in the horizontal shift register 42, to be cleared.

## Claims

1. A fluorescence imaging apparatus, comprising:

   i) excitation light irradiating means for irradiating excitation light to a measuring site, the excitation light causing the measuring site to produce fluorescence,
   ii) imaging means for imaging the fluorescence, which has been produced from the measuring site when the excitation light is irradiated to the measuring site, and
   iii) imaging control means for controlling operations of the imaging means,

   wherein the imaging means is provided with an image sensor, which comprises a plurality of pixels arrayed in two-dimensional directions and which has a fluorescence imaging region utilized for the imaging of the fluorescence and a non-imaging region other than the fluorescence imaging region, and
   the imaging control means controls such that, when signal charges are to be read from the image sensor, signal charges, which have been accumulated in at least certain pixels among pixels falling within the non-imaging region, are read with a quick reading operation, in which the signal charges are read at a reading speed higher than the reading speed for the fluorescence imaging region.

2. A fluorescence imaging apparatus, comprising:

   i) excitation light irradiating means for irradiating excitation light to a measuring site, the excitation light causing the measuring site to produce fluorescence,
   ii) imaging means for imaging the fluorescence, which has been produced from the measuring site when the excitation light is irradiated to the measuring site, and
   iii) imaging control means for controlling operations of the imaging means,

   wherein the imaging means is provided with an image sensor, which comprises a plurality of pixels arrayed in two-dimensional directions and which has a fluorescence imaging region utilized for the imaging of the fluorescence and a non-imaging region other than the fluorescence imaging region, and
   the imaging control means controls such that, when signal charges are to be read from the image sensor, signal charges, which have been accumulated in at least certain pixels among pixels falling within the non-imaging region, are read with a binning reading operation, in which the signal charges having been accumulated in a plurality of the pixels are added together, and a total sum signal charge having been obtained from the addition is read.

3. A fluorescence imaging apparatus, comprising:

   i) excitation light irradiating means for irradiating excitation light to a measuring site, the excitation light causing the measuring site to produce fluorescence,
   ii) imaging means for imaging the fluorescence, which has been produced from the measuring site when the excitation light is irradiated to the measuring site, and
   iii) imaging control means for controlling operations of the imaging means,

   wherein the imaging means is provided with an image sensor, which comprises a plurality of pixels arrayed in two-dimensional directions and which has a fluorescence imaging region utilized for the imaging of the fluorescence and a non-imaging region other than the fluorescence imaging region, and
   the imaging control means controls such that, when signal charges are to be read from the image sensor, signal charges, which have been accumulated in at least certain pixels among pixels falling within the non-imaging region, are prevented from being read.

4. A fluorescence imaging apparatus, comprising:

i) excitation light irradiating means for irradiating excitation light to a measuring site, the excitation light causing the measuring site to produce fluorescence,
ii) imaging means for imaging the fluorescence, which has been produced from the measuring site when the excitation light is irradiated to the measuring site, and
iii) imaging control means for controlling operations of the imaging means,

wherein the imaging means is provided with a charge transfer type of image sensor, which comprises a plurality of pixels arrayed in two-dimensional directions and which has a fluorescence imaging region utilized for the imaging of the fluorescence and a non-imaging region other than the fluorescence imaging region, and the imaging control means controls such that, when signal charges are to be read from the image sensor, signal charges, which have been accumulated in pixels falling within a certain area of the non-imaging region, are read with either one of a quick reading operation, in which the signal charges are read at a reading speed higher than the reading speed for the fluorescence imaging region, and a binning reading operation, in which the signal charges having been accumulated in a plurality of the pixels are added together, and a total sum signal charge having been obtained from the addition is read, and signal charges, which have been accumulated in pixels falling within the other area of the non-imaging region, are prevented from being read.

5. An apparatus as defined in Claim 3 or 4 wherein the image sensor is provided with a clearing section for clearing signal charges, which have been accumulated in pixels.

6. An apparatus as defined in Claim 3 or 4 wherein the image sensor is provided with horizontal shifting means, from which the signal charges are read in one direction,

the imaging control means controls such that the signal charges having been accumulated in the pixels are transferred to the horizontal shifting means and are then read from the horizontal shifting means, and
the fluorescence imaging region is located at a position shifted from a center position on an imaging surface of the image sensor toward a side corresponding to a read-out side of the horizontal shifting means.

7. An apparatus as defined in Claim 5 wherein the image sensor is provided with horizontal shifting means, from which the signal charges are read in one direction,

the imaging control means controls such that the signal charges having been accumulated in the pixels are transferred to the horizontal shifting means and are then read from the horizontal shifting means, and
the fluorescence imaging region is located at a position shifted from a center position on an imaging surface of the image sensor toward a side corresponding to a read-out side of the horizontal shifting means.

8. A fluorescence imaging apparatus, comprising:

i) excitation light irradiating means for irradiating excitation light to a measuring site, the excitation light causing the measuring site to produce fluorescence,
ii) imaging means for imaging the fluorescence, which has been produced from the measuring site when the excitation light is irradiated to the measuring site, and
iii) imaging control means for controlling operations of the imaging means,

wherein the imaging means is provided with a random access type of image sensor, which comprises a plurality of pixels arrayed in two-dimensional directions and which has a fluorescence imaging region utilized for the imaging of the fluorescence and a non-imaging region other than the fluorescence imaging region, and the imaging control means controls such that, when signal charges are to be read from the image sensor, only the signal charges, which have been accumulated in pixels falling within the fluorescence imaging region, are read.

# F I G . 1

100(200,300)
101
104
L2
10
109
102(202,302)
(301,201)108
106
105
L1

130(220,320)
150
134(221,321) 132 133 134 135
103b(203b,303b)
140(230,330)
11
120(210,310)
103a(203a,303a)
113 111 112 110

# F I G . 2

21
(4/3)n
24
n
25
25
22
23

# F I G . 3

n-TH LINE
.
.
.
FIFTH LINE
FOURTH LINE
THIRD LINE
SECOND LINE
26
FIRST LINE
27
28
29
22

# F I G . 4

107a    107b    106

# F I G . 5

| SIGNAL CHARGES AT NON-IMAGING REGION | SIGNAL CHARGES AT FLUORESCENCE IMAGING REGION | SIGNAL CHARGES AT NON-IMAGING REGION |

i-TH LINE

FIRST    k-TH    k'-TH    $(4/3)n$-TH

# F I G . 6

# F I G . 7

# F I G . 8

# F I G . 9